Europäisches Patentamt

(19) European Patent Office

Office européen des brevets

(11) Publication number: **0 184 314**
**B1**

(12) **EUROPEAN PATENT SPECIFICATION**

(45) Date of publication of patent specification: **11.04.90**

(21) Application number: **85307820.2**

(22) Date of filing: **29.10.85**

(51) Int. Cl.⁵: **A 61 M 29/02, A 61 M 25/00**

(54) **Rapidly inflatable balloon catheter and method.**

(30) Priority: **31.10.84 US 666873**

(43) Date of publication of application:
**11.06.86 Bulletin 86/24**

(45) Publication of the grant of the patent:
**11.04.90 Bulletin 90/15**

(84) Designated Contracting States:
**AT BE CH DE FR GB IT LI LU NL SE**

(56) References cited:
**EP-A-0 054 357**
**WO-A-83/03204**
**GB-A-1 045 202**
**GB-A-1 553 915**
**US-A-2 936 761**
**US-A-3 989 571**
**US-A-4 323 071**

(73) Proprietor: **BAXTER INTERNATIONAL INC. (a Delaware corporation)**
**One Baxter Parkway**
**Deerfield Illinois 60015 (US)**

(72) Inventor: **Ari, Suha Vakif**
**58 Eagle Run**
**Irvine California 92714 (US)**
Inventor: **Lieber, Glen Leon**
**320 Caliente Court**
**Placentia California 92670 (US)**

(74) Representative: **Geering, Keith Edwin et al**
**REDDIE & GROSE 16 Theobalds Road**
**London WC1X 8PL (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

### Background of the invention

In percutaneous transluminal angioplasty, a dilation catheter is inserted into an artery to dilate the artery. For this purpose, the typical dilation catheter has an inflatable balloon adjacent its distal end and a balloon inflation lumen through which the balloon is inflated and deflated. Inflation is accomplished by forcing an appropriate inflation liquid through the lumen to the balloon, and deflation is accomplished by withdrawing the liquid from the balloon. Inflation of the balloon dilates the adjacent regions of the artery to provide lower restriction to blood flow through the artery.

In carrying out the dilation procedure, it is necessary to repeatedly inflate and deflate the balloon to repeatedly dilate the artery. During the time that the balloon is inflated, blood flow through the artery is blocked. For this reason, it is necessary that the inflation and deflation of the balloon be carried out as rapidly as possible. This is difficult to do because the balloon inflation lumen is long and of small diameter, and consequently, the balloon inflation lumen presents a substantial resistance to the flow of the inflation liquid to and from the balloon.

One other complicating factor is that air must be purged from the balloon before the dilation catheter is used for its intended purpose. This can be accomplished, for example, by inserting a vent tube through the balloon inflation lumen into the balloon. A purging liquid is then introduced through the balloon inflation lumen, and the air within the balloon is vented through the vent tube. One example of such an arrangement is shown in US—A—4,323,071. One problem with this procedure is that the insertion of the vent tube must be carefully carried out not to damage the balloon and may be cumbersome if the vent tube is attempted to be taken out to obtain a larger inflation lumen.

US—A—2,936,761 discloses a catheter having proximal and distal ends, at least first and second lumens and a balloon adjacent the distal end, with the first and second lumens extending from the proximal end to the interior of the balloon. In use of this catheter sterilising fluid is removed by passing a gaseous medium through one lumen and out through the other lumen. In one embodiment in which the two lumens are united in a terminal tube portion at the proximal end, inflation of the balloon is achieved by inserting a long Luer needle through the terminal tube into one lumen while the other lumen is closed. Clamps may be used to close the lumens.

### Summary of the invention

This invention provides a dilation catheter and a method for sequentially purging, inflating and deflating the balloon of a catheter outside the human or animal body which eliminate the need for vent tube insertion and the use of Luer needles to introduce fluid into the balloon lumens. In addition, with this invention, the balloon of the dilation catheter can be rapidly inflated and deflated so that useful dilation pressure can be applied for the maximum length of time.

This invention provides a catheter assembly adapted to dilate a tubular body member, such as an artery, which comprises a catheter having distal and proximal ends, means for defining at least first and second lumens, and a balloon adjacent the distal end with the first and second lumens extending to the interior of the balloon.

According to the invention a connector is provided at the proximal end of the catheter and the assembly includes first and second means interchangeably connectable to the connector, the first means being means for introducing a purging liquid to the first lumen such that the purging liquid flows in series through the first lumen, the balloon and the second lumen to purge air from the balloon out through the second lumen; and the second means being means for introducing an inflation liquid to the balloon through both the first and second lumens to the balloon to inflate the balloon and for discharging the inflation liquid from the balloon through both the first and second lumens to deflate the balloon.

The catheter assembly preferably also includes a through lumen that may be used, for example, for a guide wire, monitoring of pressure distally of the balloon, and the infusion of a contrast medium or medication.

The invention also provides a method for sequentially purging, inflating and deflating the balloon of a catheter outside the human or animal body.

To purge air from the balloon, the first and second lumens are used in series. More specifically, a purging liquid is passed in series through the first lumen, the balloon and the second lumen to purge air from the balloon out through the second lumen. Thus, no insertable vent tube is required.

Thereafter, the first and second lumens are used in parallel for inflating and/or deflating of the balloon. Preferably, the first and second lumens are used in parallel for both inflation and deflation of the balloon. This is accomplished by passing an inflation liquid through both of the first and second lumens to inflate the balloon and discharging the inflation liquid from the balloon through both of the first and second lumens.

The purging of air from the balloon is preferably carried out with the balloon pointed downwardly. Following the purging cycle, the catheter can be inserted into the vascular system of the patient to dilate a region of the vascular system.

The catheter assembly includes means for introducing the purging liquid, means for introducing the inflation liquid and means for discharging the inflation liquid from the balloon. Although these means can be of various different constructions, the catheter includes a connector located proximally of the balloon, and the means for introducing the purging liquid is removably coupled to the connector. This permits such means to be removed from the connector and replaced with the means for introducing the inflation liquid.

In a preferred construction, the means for introducing the purging liquid includes a purge fitting having a first leg communicating with the first lumen at the connector and a second leg receiving the second lumen. The first leg is communicable with a source of the purging liquid. The purge fitting can be removed and replaced with, for example, a syringe which can introduce the inflation liquid and enable discharge of the inflation liquid from the balloon.

According to a preferred embodiment of the invention the catheter includes a catheter body and an extension tube coupled to the catheter body in communication with a through lumen in the catheter body and having a passage therethrough which forms an extension of the through lumen. The balloon has an inflatable portion in communication with the balloon inflation lumens, attachment portions on opposite sides of the inflatable portion sealed to the catheter body and the tube, respectively, and an at least somewhat flexible tubular distal portion extending distally beyond the tube to form an extension of the through lumen and to define the distal end of the catheter. The tubular distal portion is preferably of smaller cross-sectional area than the tube and is smoothly blended into such smaller cross-sectional area to avoid the presence of any sharp or abrupt corners or shoulders. Preferably, the tubular distal portion is more flexible than regions of the catheter body adjacent the tube. This facilitates insertion and movement of the catheter into and through the vascular system and minimizes the likelihood of damage to the tissue.

The invention, together with additional features and advantages thereof, may best be understood by reference to the following description taken in connection with the accompanying illustrative drawing.

Brief description of the drawing

Fig. 1 is an elevational view of a catheter assembly constructed in accordance with the teachings of this invention.

Fig. 2 is a partially schematic, longitudinal sectional view through the purge fitting and the associated connector.

Figs. 3 and 4 are enlarged sectional views taken generally along lines 3—3 and 4—4 of Fig. 1, respectively.

Fig. 5 is an enlarged, fragmentary sectional view taken on an axial plane and showing the distal end portion of the catheter.

Fig. 6 is a fragmentary sectional view taken generally along line 6—6 of Fig. 5.

Figs. 7 and 8 are somewhat schematic views partially in section illustrating the purging and inflation cycles, respectively. The proximal through lumen extension is not shown in Figs. 7 and 8.

Description of the preferred embodiment

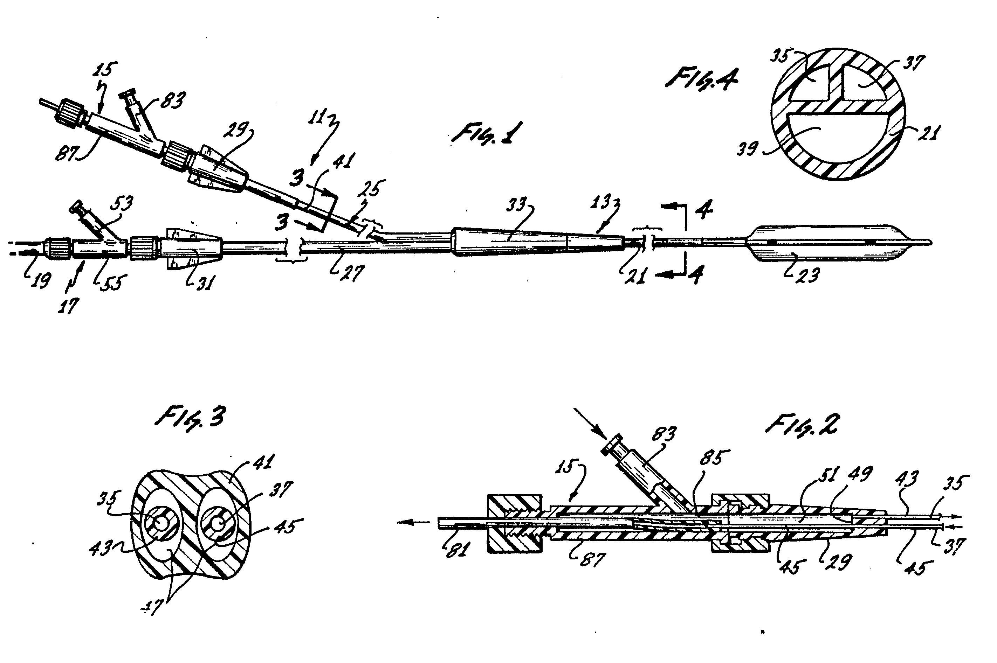

Fig. 1 shows a catheter assembly 11 which includes a catheter 13, a purge fitting 15 and a Y-fitting 17, both of which are coupled to the catheter. The catheter assembly also includes a conventional guidewire introducer 19 partially within the proximal end of the Y-fitting 17.

The catheter 13 includes a catheter body 21, a balloon 23, a vent-inflation extension 25, a through lumen extension 27 and connectors 29 and 31 which define the proximal end of the catheter 13 coupled to the proximal ends of the extensions 25 and 27, respectively. The catheter 13 also includes a sleeve 33 for encasing portions of the extensions 25 and 27 and the catheter body 21.

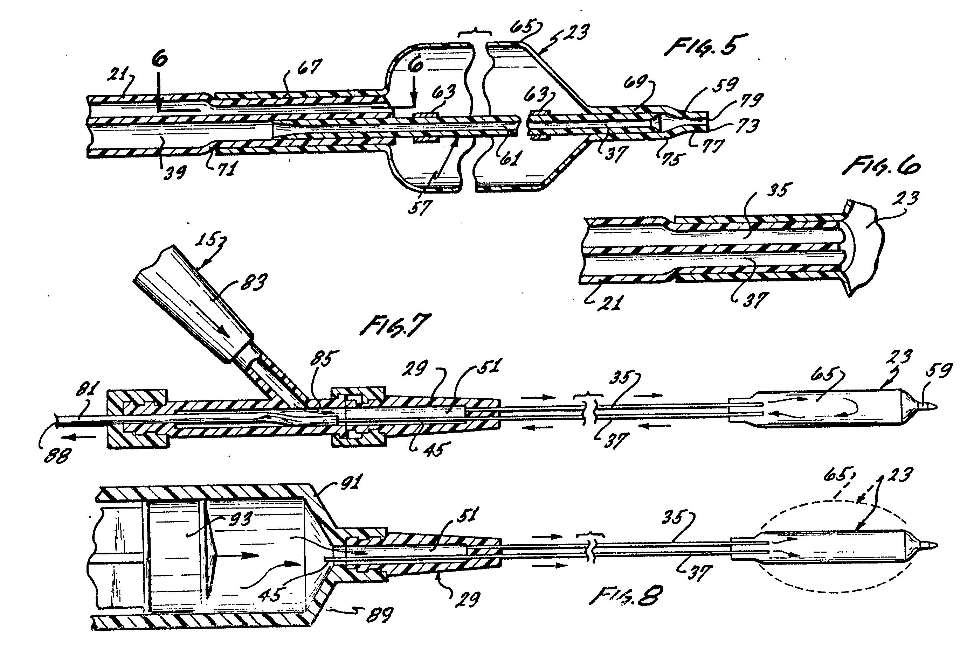

The catheter body 21 may be in the form of an elongated, flexible, cylindrical tube (Fig. 4) of a suitable plastic material having suitable internal partitions defining portions of balloon lumens 35 and 37 and a through lumen 39. The balloon lumens 35 and 37 extend from the proximal end of the catheter body 21 to the interior of the balloon 23 at the distal end of the catheter body 21 as shown in Figs. 5 and 6. The balloon lumens 35 and 37 are extended proximally of the catheter body 21 by the extension 25 which comprises an outer tube 41 (Fig. 3) and inner tubes 43 and 45 extending through parallel passages 47 of the outer tube. The outer tube 41 extends within the sleeve 33, and the inner tubes 43 and 45 are received within the lumens 35 and 37 of the catheter body 21 and suitably retained therein. Similarly, the connector 29 is suitably mounted on the outer tube 41. As shown in Fig. 2, the inner tube 43 terminates at an inner face 49 of the connector 29, and the inner tube 45 extends completely through a passage 51 in the connector and completely through the connector.

The through lumen 39 is extended proximally by the extension 27 which extends into the sleeve 33 and a proximal portion of the through lumen 39 in the catheter body 21. The sleeve 33 surrounds the extensions 25 and 27 and the catheter body 21 in a known manner. The Y-fitting 17, which is releasably coupled to the connector 31, has a leg 53 which is open to the through lumen 39 to permit the obtaining of blood pressure or blood samples, the infusion of medication, etc. The Y-fitting 17 has a second leg 55 in which the guidewire introducer 19 is mounted to permit insertion of a guidewire (not shown) through the through lumen 39.

The through lumen 39 is extended distally by a tube 57 and a tubular distal portion 59 of the balloon 23 as shown by way of example in Fig. 5. Although various constructions are possible, the tube 57 is received within a distal end portion of the lumen 39 of the catheter body 21 and is retained therein in any suitable manner, such as by an adhesive or heat sealing. The tube 57 is elongated and flexible and may be constructed of a suitable plastic material. The tube 57 has an axial passage 61 extending through it which forms an extension of the through lumen 39.

Preferably, radiopaque bands 63 are suitably retained on the tube 57 within the balloon 23 to permit the physician to ascertain the location of the balloon when it is in the patient's vascular system.

The balloon 23, which is adjacent the distal end of the catheter body 21, has an inflatable portion 65, attachment portions 67 and 69 on opposite sides of the inflatable portion 65 which are sealed to the catheter body 21 and the tube 57, respectively, and the tubular distal portion 59. The balloon 23, which is constructed of a suitable plastic, such as polyethylene, may have the attachment portions 67 and 69 shrunk onto the catheter body 21 and the tube 57. By tightly shrinking the attachment portion 67 onto the catheter body 21, the catheter body may be compressed somewhat radially inwardly to form an inwardly extending annular shoulder 71 which tends to make the periphery of the attachment portion 67 approximately coextensive with the periphery of the catheter body 21. This minimizes changes in cross section along the length of the catheter 13.

The tube 57 extends completely through the inflatable portion 65, which is of larger cross-sectional area than the tube and the catheter body 21. However, the tube 57 does not project completely through the balloon 23 in that the tubular distal portion 59 extends distally of the tube 57 to form the distal end 73 of the catheter 13. The tubular distal portion 59 is of smaller cross section than the tube 57 and is smoothly and gradually blended into such smaller cross section by an inclined annular wall section 75 which extends distally from the distal end of the tube 57. The tubular distal portion 59 terminates distally in a cylindrical section 77 which defines a distal port 79 at the distal end 73.

The balloon 23 is integrally constructed, and the tubular distal portion 59 is resiliently flexible. Preferably, the tubular distal portion 59 is more flexible than regions of the catheter body 21 adjacent the tube 57.

First means is provided for introducing a purging liquid to the lumen 35. Although this means can take different forms, in the embodiment illustrated, it includes the purge fitting 15 and a vent tube 81 carried by the purge fitting. As shown in Fig. 2, the purge fitting 15 is removably coupled to the connector 29 in a conventional manner. The purge fitting 15 is in the form of a Y-fitting having a first leg 83 in communication with the lumen 35 through a common passage 85 and a passage 51 of the connector 29. The inner tube 45, which defines the proximal extension of the lumen 37, extends completely through the passage 51 of the connector 29 and into the passage 85 of a second leg 87 of the purge fitting 15. The vent tube 81 is slidably received within the passage of the second leg 87, and the vent tube slidably receives the proximal end of the inner tube 45 as shown in Fig. 2. The vent tube 81 terminates in a vent opening 88.

With the purge fitting 15 coupled to the connector 29, the catheter assembly 11 can be used to purge air from the balloon 23 as shown in Fig. 7. The first leg 83 is coupled to a source of the purging liquid (not shown), and the distal end of the balloon is pointed downwardly. The purging liquid is then passed in series through the first leg 83, the passages 85 and 51, the lumen 35, the inflatable portion 65 of the balloon 23, the lumen 37 and the vent tube 81. The passing of the purging liquid in series through the lumens 35 and 37 forces the air in the balloon out through the lumen 37 and the vent tube 81 to the atmosphere. The purging liquid is passed in series through the lumens 35 and 37 in this fashion at least until the purging liquid emerges from the vent opening 88. At this time, it is known that all of the air has been purged from the balloon 23.

Means is also provided for introducing an inflation liquid to the balloon 23 and for discharging the inflation liquid from the balloon. Although this means can take different forms, in the embodiment illustrated, it includes a syringe 89 (Fig. 8) for accomplishing both of these purposes. More specifically, the purge fitting 15 is removed from the connector 29, and the syringe 89 is attached to the proximal end of the connector. The vent tube 81 can be easily removed with the purge fitting 15 by pulling if off the proximal end of the inner tube 45. The syringe 89 includes the usual barrel 91 and plunger 93 which is movable to the right (as viewed in Fig. 8) on a discharge stroke and to the left (as viewed in Fig. 8) in a suction stroke.

With the syringe 89 charged with inflation liquid, the plunger 93 on the discharge stroke introduces an inflation liquid in parallel through both of the lumens 35 and 37 to inflate the balloon as shown in dashed lines in Fig. 8. More specifically, the inflation liquid from the barrel 91 is forced through the passage 51 to the lumen 35 and forced directly from the barrel into the inner tube 45. Similarly, by moving the plunger 93 on the suction stroke, the inflation liquid is discharged from the balloon 23 in parallel through both of the lumens 35 and 37 to deflate the balloon and move the inflation liquid back into the barrel 91. Because both of the lumens 35 and 37 are used in parallel to inflate and deflate the balloon 23, inflation and deflation of the balloon takes place very rapidly.

In use of the catheter assembly 11, air is first purged from the balloon 23 as described above. Next, the catheter is inserted into the vascular system of the patient using known techniques to place the balloon 23 in the region of the vascular system, such as an appropriate artery, which is to be dilated. After the purge fitting 15 has been replaced with the syringe 89, the balloon 23 is inflated as described above to dilate the desired region of the vascular system and then deflated to discontinue dilation. Inflation and deflation of the balloon 23 are carried out rapidly. The dilation procedure may be carried out repeatedly within the artery before the catheter is removed from the patient's vascular system. The purging liquid and the inflation liquid may be the same liquid and may be any of a variety of known solutions, such as a mixture of Renographen and saline.

During the insertion of the catheter 13, the tubular distal portion 59, being of small diameter, passes through the artery with relative ease. The inclined wall section 75 provides essentially no shoulder that could cause damage to the tissue. In addition, the flexible nature of the tubular distal portion 59 further minimizes likelihood of injury to the tissue.

## Claims

1. A catheter assembly (11) for dilation of a tubular body member comprising:
a catheter (13) having a distal end, a proximal end, at least first and second lumens (35, 37), a balloon (23) adjacent the distal end with the first and second lumens (35, 37) extending from the proximal end to the interior of the balloon (23); characterised by:
a connector (29) at said proximal end with said first and second lumens (35, 37) terminating in said connector (29);
first and second means interchangeably connecatable to the connector (29);
said first means (15) being means for introducing a purging liquid to the first lumen (35) such that the purging liquid flows in series through the first lumen (35), the balloon (23) and the second lumen (37) to purge air from the balloon out through the second lumen (37); and
said second means (89) being means for introducing an inflation liquid to the balloon (23) through both the first and second lumens (35, 37) to inflate the balloon (23) and for discharging the inflation liquid from the balloon (23) through both the first and second lumens (35, 37) to deflate the balloon (23).

2. A catheter according to claim 1 wherein the first means (15), includes a vent tube (81) for slidably receiving a proximal extension of the second lumen (37) when the first means (15) is mounted on the connector (29) whereby the purging liquid can be vented through the vent tube (81).

3. A catheter assembly according to claim 1, wherein said second means includes a syringe (89) mountable on the connector (29) said syringe (89) having pumping and suction strokes, and, in use, said syringe (89) introducing inflation liquid to both first and second lumens (35, 37) on the pumping stroke and withdrawing inflation liquid from the balloon (23) through both first and second lumens (35, 37) on the suction stroke.

4. A catheter assembly according to any one of claims 1 to 3, wherein the catheter (13) includes a catheter body (21) through which said first and second lumens (35, 37) and a third lumen (39) extend and an extension tube (57) coupled to the catheter body (21) in communication with the third lumen (39), and having a passage (61) therethrough which forms an extension of the third lumen (39), said extension tube (57) extending partially through the balloon (23), and said balloon has an inflatable portion (65) communicating with the first and second lumens (35, 37), attachment portions (67, 69) on opposite sides of the inflatable portion (65) and sealed to the catheter body (21) and the tube (57), respectively, and a tubular distal portion (59) extending distally beyond the tube (57) to form an extension of the third lumen (39) and to define the distal end of the catheter.

5. A catheter according to claim 4, wherein said tubular distal portion (59) is more flexible than regions of the cathether body (21) adjacent the tube (57).

6. A catheter according to claim 4 or claim 5, wherein the tubular distal portion (59) is of smaller cross-section than the tube (57) and the catheter smoothly blends into such smaller cross-section.

7. A catheter according to any one of claims 4 to 6, wherein said extension tube (57) is partly received within the distal portion of the third lumen (39) in the catheter body (21).

8. A method for sequentially purging, inflating and deflating the balloon of a catheter (13) outside the human or animal body, said catheter (13) having a distal end, a proximal end, at least first and second lumens (35, 37), a balloon (23) adjacent the distal end with the first and second lumens (35, 37) extending from the proximal end to the interior of the balloon (23) and a connector (29) at said proximal end, the first and second lumens (35, 37) terminating in said connector (29), which comprises the steps of:
attaching to the connector (29) first means (15) for purging air from the balloon (23);
introducing purging liquid from the first means (15), in series through the first lumen (35), the balloon (23) and the second lumen (37) to purge air from the balloon (23) through the second lumen (37);
detaching the first means (15) from the connector (29);
attaching to the connector (29) second means (83) for introducing inflation liquid to and removing inflation liquid from the balloon (23);
introducing inflation liquid through both the first and second lumens (37) to inflate the balloon (23); and
subsequently discharging the inflation liquid from the balloon (23) through both the first and second lumens (35, 37) to deflate the balloon.

## Patentansprüche

1. Katheteranordnung (11) zur Dilation eines rohrförmigen Körperteils, die aufweist:
einen Katheter (13), der ein distales Ende, eine proximales Ende, wenigstens erste und zweite Lumen (35, 37) und einen Ballon (23) in der Nähe des distalen Endes mit den ersten und zweiten Lumen (35, 37) hat, die sich vom proximalen Ende in das Innere des Ballons (23) erstrecken, gekennzeichnet durch:
einen Verbinder (29) am proximalen Ende mit den ersten und zweiten Lumen (35, 37), die in dem Verbinder (29) enden, und

erste und zweite Einrichtungen, die mit dem Verbinder (29) austauschbar verbindbar sind,

wobei die erste Einrichtung (25) eine Einrichtung zum Einleiten einer Reinigungsflüssigkeit in das erste Lumen (35) derart ist, daß die Reinigungsflüssigkeit hintereinander durch das erste Lumen (35), den Ballon (33) und das zweite Lumen (37) strömt, um Luft aus dem Ballon über das zweite Lumen (37) zu entfernen, und

wobei die zweite Einrichtung (89) eine Einrichtung zum Einleiten einer Aufblasflüssigkeit in den Ballon (23) über beide erste und zweiten Lumen (35, 37) ist, um den Ballon (23) aufzublasen, und wobei die Einrichtung die Aufblasflüssigkeit von dem Ballon (23) über beide erste und zweite Lumen (35, 37) abgibt, um den Ballon (23) abzulassen.

2. Katheter nach Anspruch 1, bei dem die erste Einrichtung (15) ein Entlüftungsrohr (81) zum gleitbaren Aufnehmen einer proximalen Verlängerung des zweiten Lumens (37) enthält, wenn die erste Einrichtung (15) an dem Verbinder (29) angebracht ist, wodurch die Reinigungsflüssigkeit über das Entlüftungsrohr (81) abgelassen werden kann.

3. Katheteranordnung nach Anspruch 1, bei der die zweite Einrichtung eine Spritze (89) enthält, die an dem Verbinder (29) anbringbar ist, wobei die Spritze (89) Pump- und Saughübe hat und im Gebrauchszustand die Spritze (89) Aufblasflüssigkeit in die beiden ersten und zweiten Lumen (35, 37) beim Pumphub einleitet und die Aufblasflüssigkeit von dem Ballon (23) über die beiden ersten und zweiten Lumen (35, 37) beim Saughub abzieht.

4. Katheteranordnung nach einem der Ansprüche 1 bis 3, bei der der Katheter (13) einen Katheterkörper (21) umfaßt, durch den die ersten und zweiten Lumen (34, 37) und ein drittes Lumen gehen, wobei ein Verlängerungsrohr (57) mit dem Katheterkörper (21) in kommunizierender Verbindung mit dem dritten Lumen (39) verbunden ist und einen durch dasselbe gehenden Durchgang (61) hat, der eine Verlängerung des dritten Lumens (39) bildet, das Verlängerungsrohr (57) teilweise durch den Ballon (23) geht und der Ballon einen aufblasbaren Abschnitt (65), der in kommunizierender Verbindung mit den ersten und zweiten Lumen (35, 37) steht, Befestigungsabschnitte (67, 69) auf gegenüberliegenden Seiten des aufblasbaren Abschnitts (65) und gegenüber dem Katheterkörper (21) und dem Rohr (57) jeweils abgedichtet und einen rohrförmigen, distalen Abschnitt (59) hat, der sich distal über das Rohr (57) hinaus erstreckt, um eine Verlängerung des dritten Lumens (39) und das distale Ende des Katheters zu bilden.

5. Katheter nach Anspruch 4, bei dem der rohrförmige, distale Abschnitt (59) flexibler als Bereiche des Katheterkörpers (21) in der Nähe des Rohrs (57) ist.

6. Katheter nach Anspruch 4 oder Anspruch 5, bei dem der rohrförmige, distale Abschnitt (59) einen kleineren Querschnitt als das Rohr (57) hat und der Katheter gleichförmig in diesen kleineren Querschnitt übergeht.

7. Katheter nach einem der Ansprüche 4 bis 6, bei dem das Verlängerungsrohr (57) teilweise in dem distalen Abschnitt des dritten Lumens (39) in dem Katheterkörper (21) aufgenommen ist.

8. Verfahren zum sequentiellen Reinigen, Aufblasen und Ablassen des Ballons eines Katheters (13) außerhalb des menschlichen oder tierischen Körpers, wobei der Katheter (13) ein distales Ende, ein proximales Ende, wenigstens erste und zweite Lumen (35, 37), einen Ballon (23) in der Nähe des distalen Endes mit den ersten und zweiten Lumen (35, 37), die sich von dem proximalen Ende in das Innere des Ballons (23) erstrecken, und einen Verbinder (29) am proximalen Ende hat, wobei die ersten und zweiten Lumen (35, 37) in dem Verbinder (29) enden, wobei das Verfahren folgende Schritte aufweist:

Anbringen einer ersten Einrichtung (15) zum Entfernen von Luft aus dem Ballon (23) an dem Verbinder (29),

Einleiten einer Reinigungsflüssigkeit von der ersten Einrichtung (15) hintereinander durch das erste Volumen (35), den Ballon (23) und das zweite Volumen (37), um Luft aus dem Ballon (23) über das zweite Volumen (37) zu entfernen,

Abnehmen der ersten Einrichtung (15) von dem Verbinder (29),

Anbringen einer zweiten Einrichtung (83) zum Einleiten einer Aufblasflüssigkeit in den Ballon (23) und zum Ableiten der Aufblasflüssigkeit von dem Ballon (23) an dem Verbinder (29),

Einleiten einer Aufblasflüssigkeit durch beide erste und zweite Lumen (37), um den Ballon (23) aufzublasen, und abschließendes Ausleiten der Aufblasflüssigkeit aus dem Ballon (23) über die beiden ersten und zweiten Lumen (35, 37), um den Ballon abzulassen.

**Revendications**

1. Assemblage d'un cathéter (11) pour la dilatation d'un organe tubulaire du corps comprenant:

un cathéter (13) ayant une extrémité distale, une extrémité proximale, au moins des premier et second passages (35, 37), un ballon (23) adjacent à l'extrémité distale avec les premier et second passages (35, 37) s'étendant de l'extrémité proximale jusqu'à l'intérieur du ballon (23); caractérisé par:

un connecteur (29) à ladite extrémité proximale avec lesdits premier et second passages (35, 37) se terminant dans ledit connecteur (29);

des premier et second moyens pouvant être connectés de manière interchangeable au connecteur (29);

ledit premier moyen (15) étant un moyen pour introduire un liquide de purge dans le premier passage (35) de manière que le liquide de purge s'écoule en série à travers le premiere passage (35), le ballon (23) et le second passage (37) pour purger l'air du ballon vers l'extérieur à travers le second passage (37); et

ledit second moyen (89) étant un moyen pour introduire un liquide de gonflage dans le ballon (23) à travers les premier et second passages (35, 37) pour gonfler le ballon (23) et pour refouler le liquide de gonflage du ballon (23) à travers les premier et second passages (35, 37) pour dégonfler le ballon (23).

2. Cathéter selon la revendication 1 où le premier moyen (15) comprend un tube d'évent (81) pour recevoir de manière coulissante une extension proximale du second passage (37) quand le premier moyen (15) est monté sur le connecteur (29) pour qu'ainsi le liquide de purge puisse être éventé à travers le tube d'évent (81).

3. Assemblage d'un cathéter selon la revendication 1, où ledit second moyen comprend une seringue (89) pouvant être montée sur le connecteur (29), ladite seringue (89) ayant des courses de pompage et d'aspiration et, en utilisation, ladite seringue (89) introudisant un liquide de gonflage dans des premier et second passages (35, 37) lors de la course de pompage et retirant le liquide de gonflage du ballon (23) à travers lesdits premier et second passages (35, 37) lors de la course d'aspiration.

4. Assemblage d'un cathéter selon l'une quelconque des revendications 1 à 3, où le cathéter (13) comprend un corps de cathéter (21) à travers lequel s'étendent lesdits premier et second passages (35, 37) et un troisième passage (39) et un tube en extension (57) couplé au corps (21) du cathéter en communication avec le troisième passage (39) et traversé d'un conduit (61) qui forme une extension du troisième passage (39), ledit tube en extension (57) s'étendant partiellement à travers le ballon (23) et ledit ballon a une portion gonflable (65) communiquant avec les premier et second passages (35, 37), des portions de fixation (67, 69) sur des côtés opposés de la portion gonflable (65) et scellées au corps (21) du cathéter et au tube (57), respectivement, et une portion distale tubulaire (59) s'étendant à l'extrémité distale au delà du tube (57) pour former une extension du troisième passage (39) et définir l'extrémité distale du cathéter.

5. Cathéter selon la revendication 4, où ladite portion distale tubulaire (59) est plus flexible que des régions du corps (21) du cathéter adjacentes au tube (57).

6. Cathéter selon la revendication 4 ou la revendication 5, où la portion distale tubulaire (59) est de plus petite section transversale que le tube (57) et le cathéter se confond doucement avec cette plus petite section transversale.

7. Cathéter selon l'une quelconque des revendications 4 à 6, où ledit tube en extension (57) est partiellement reçu dans la portion distale du troisième passage (39) dans le corps (21) du cathéter.

8. Méthode pour séquentiellement purger, gonfler et dégonfler le ballon d'un cathéter (13) en dehors du corps humain ou animal, ledit cathéter (13) ayant une extrémité distale, une extrémité proximale, au moins des premier et second passages (35, 37), un ballon (23) adjacent à l'extrémité distale avec les premier et second passages (35, 37) s'étendant de l'extrémité proximale jusqu'à l'intérieur du ballon (23) et un connecteur (29) à ladite extrémité proximale, les premier et second passages (35, 37) se terminant dans ledit connecteur (29), qui comprend les étapes de:

attacher au connecteur (29) un premier moyen (15) pour purger l'air du ballon (23);

introduire le liquide de purge du premier moyen (15), en série à travers le premier passage (35), le ballon (23) et le second passage (37) pour purger l'air du ballon (23) à travers le second passage (37);

détacher le premier moyen (15) du connecteur (29);

attacher au connecteur (29) un second moyen (83) pour introduire un liquide de gonflage vers et enlever le liquide de gonflage du ballon (23);

introduire un liquide de gonflage à travers les premier et second passages (37) pour gonfler le ballon (23); et

refouler subséquemment le liquide de gonflage du ballon (23) à travers les premier et second passages (35, 37) pour dégonfler le ballon.

Fig. 4

Fig. 1

Fig. 3

Fig. 2

EP 0 184 314 B1

Fig.5 Fig.6 Fig.7 Fig.8